Europäisches Patentamt

European Patent Office

Office européen des brevets

(11) Publication number: **0 150 905**
A2

# EUROPEAN PATENT APPLICATION

) Application number: 85300059.4

) Date of filing: 04.01.85

(51) Int. Cl.⁴: **G 01 N 33/542,** G 01 N 33/563, G 01 N 33/577

(30) Priority: 05.01.84 US 568556

(43) Date of publication of application: 07.08.85
Bulletin 85/32

(84) Designated Contracting States: DE FR GB

(71) Applicant: ORTHO DIAGNOSTIC SYSTEMS INC., One Johnson & Johnson Plaza, New Brunswick New Jersey 08933 (US)

(72) Inventor: Hansen, W. Peter, 49 Cedar Street, Middlesboro Massachusetts 02346 (US)
Inventor: Chan, Joseph L.W., 138 Colonial Drive, Quincy Massachusetts 02169 (US)
Inventor: IP, Stephen H.C., 3 Heather Lane, Mansfield Massachusetts 02048 (US)

(74) Representative: Jones, Alan John et al, CARPMAELS & RANSFORD 43 Bloomsbury Square, London, WC1A 2RA (GB)

(54) Anti-idiotype assay using fluorescent energy transfer.

(57) Homogeneous immunoassay for the detection of an analyte based upon competition between the analyte and an anti-idiotype antibody for a binding site on an antibody specific for the analyte. Detection is accomplished by measuring the changes in flurescent emission of either the donor or acceptor molecule of a donor-acceptor fluorescent energy transfer pair or changes in the donor fluorescent emission time constant.

# ANTI-IDIOTYPE ASSAY USING FLUORESCENT ENERGY TRANSFER

## Field of the Invention

This invention relates generally to immunoassay methods useful for detecting soluble substances or analytes such as antigens and more specifically, relates to homogeneous assays relying upon the detection of changes in distances or orientation between two different types of antibodies based upon the immunological reaction of one of the antibodies.

The detection of specified antigens (defined as a substance whose introduction into an animal stimulates the production of antibodies capable of reacting specifically therewith), haptens (a substance requiring additional accessory materials before its introduction into an animal stimulates the production of antibodies specific therefor), and the like substances (hereinafter collectively referred to as analytes) in body fluids such as blood, sputum, urine, and the like has in recent years become of utmost importance in both the research and clinical environment. The detection of analytes, particularly antigens, and antibodies can often be related to various disease states and consequently is of extreme usefulness in diagnosis as well as gaining basic understandings concerning the genesis of disease as well as monitoring the effectiveness of therapies therefor.

Consequently, improved methods for detecting analytes in aqueous samples are constantly sought. In particular, assays are typically characterized by their speed and facility of employment as well as by their sensitivity. Preferred assays are those requiring less effort to perform in shorter time periods and characterized by greater sensitivity.

In one class of assays known as inhomogeneous assays, the sample containing the substance to be detected is mixed with the reactants

0150905

and after permitting the immunological reactions to occur, the resultant constituents are separated into soluble and insoluble phases and one or the other analyzed for the presence of a detectable label. Such inhomogeneous assays disadvantageously require separation and washing steps as well as accessory materials and apparatus to accomplish these steps.

It is an object of the present invention to avoid these disadvantages by providing a homogeneous immunoassay which does not require a separation step prior to detection of the label.

Immunoassays in general are based upon the immunological reaction between proteins such as antibodies, antibody fragments or even artificially generated peptides (hereinafter collectively referred to as binding partners) and the substance for which they are specific. These substances shall hereafter be generally referred to as analytes. Immunological reactions are generally characterized by their high specificity and accordingly numerous schemes have been developed in order to take advantage of this characteristic. Typically, such schemes require either purified antigen to compete with the antigen being measured and a labeled and immobilized antibody, or multiple immobilized and labeled antibodies reactive between themselves and the antigen. Those techniques which require purified antigen to compete with sample antigen for the binding site on the antibody disadvantageously entail difficult and expensive manufacturing processes in order to produce the purified antigen in a form that is sufficiently intact as to link with a specific binding partner. This may be particularly difficult if the antigen is a cell surface receptor.

It is an object of the present invention to provide a competitive assay which does not rely upon competition with a purified antigen or analyte and thus does not require difficult and expensive manufacturing processes heretofore associated with the production of purified intact antigen.

CRL-5

Nussenzweig et al. described in Science, 215:1637-1639 (1982) a competitive solid-phase radioimmunoassay method which does not rely upon competition of the sample antigen with a purified, investigator supplied antigen reagent. Instead, Nussenzweig describes the use of an anti-idiotype antibody. An idiotype may be defined as an individually specific antigenic determinant associated with a particular type of antibody but not with other members of the general antibody class and for which an antibody can be raised capable of specifically reacting with the idiotype. For instance, the idiotype is generally thought of as associated with the variable region of an antibody and may also comprise whole or part of the antigen binding site. In contrast, allotypic determinants are determinants commonly associated with all members of the class and would include, for instance, the Fc portions or nonvariable regions of antibodies.

It is an object of the present invention to utilize an anti-idiotype antibody approach to thereby benefit from the value that such antibodies may provide.

Heretofore, anti-idiotype antibodies have been generally used in the investigations of immune functions and how such systems can break down creating auto-immune diseases. References regarding these aspects include the following: Brient et al., "Quantitative Investigations of Idiotypic Antibodies", J. Exp. Med., 132:951-962 (1970); Geha et al., "Anti-Idiotypic Antisera in Man", J. Immunology, Vol. 121, 4:1518-1523 (1978); and Rauch et al., "A High Frequency Idiotypic Marker of Anti-DNA Autoantibodies in MRL-Ipr/Ipr Mice", J. Immunology, Vol. 129, 1:236-241 (1982).

It is another object of the present invention to provide methods of using anti-idiotype antibodies or binding partners in immunoassays thereby greatly expanding their utilitiy.

All immunoassays have, as one common characteristic, the requirement that the immunological reaction be detectable. Typically this is accomplished by associating with either 1) the antigen or analyte for which the antibody is specific, or 2) the antibody itself, a label which may be readily detected in either the aqueous or solid phases. Such labels may include, for instance, fluorophores, phosphorescent molecules, radioisotopes, enzymes, reflective particles and the like. Although all of these labels and the technology associated with their detection have over recent years become highly developed, they typically require the removal of the labeled constituent which has not participated in the immunological reaction.

It is an object of the present invention to rely instead upon yet another class of labels, specifically, those which exhibit fluorescent energy transfer characteristics.

Fluorescence energy transfer occurs between two molecules, one of which is deemed a donor and the other called an acceptor. Typically, the donor molecule is excited by energy of one wavelength (actually a bell-shaped spectrum of wavelengths having a peak or optimum wavelength) and exhibits a fluorescence energy emission curve much like that of the typical fluorophore. The acceptor molecule is chosen to have an excitation wavelength and preferably a significant portion of its excitation spectrum which falls within the emission wavelength spectrum of the donor molecule. Under optimum conditions, the donor peak emission wavelength will be approximately equal to the acceptor peak excitation wavelength. Acceptance by the acceptor molecule of the energy from the donor molecule results in diminished fluorescence of the donor molecule. The acceptor molecule in turn may be a chromophore, i.e. a molecule exhibiting no innate fluorescence, but will preferably be a fluorescent molecule having its own characteristic fluorescence emission spectra.

Energy transfer in a true fluorescence energy transfer pair takes place by a dipole-dipole energy transfer process whose efficiency varies with the inverse 6th power of the distance between the donor

CRL-5

and acceptor molecules. Thus, changes in fluorescent energy emission may be used to determine proximity relationships between acceptor and donor molecules or molecules to which they are attached. Further, the dipole-dipole energy transfer process, pursuant to Forster's theory, also has an orientation component which affects transfer efficiency. These and other fluorescence energy transfer considerations have been described in a useful reference written by Lubert Stryer entitled "Fluorescence Energy Transfer as a Spectroscopic Ruler", Annuals Review Biochem., 47:819-846 (1978).

It is an object of the present invention to utilize the proximity and orientation sensitivity between donor and acceptor molecules in a competitive, homogeneous immunoassay.

The use of a fluorescence energy transfer labeling technique has been described by Ullman et al. in U.S. Patent Nos. 4,199,559; 3,996,345; 4,174,384; and 4,261,968. Ullman teaches the employment of a fluorescer-quencher pair of molecules wherein the fluorescer molecule is excited by light of a specified wavelength and fluoresces at a longer wavelength. The presence of a quencher molecule in close proximity to the fluorescer, however, results in the quenching of this fluorescence thereby contributing to a decrease in measurable fluorescence. Accordingly, Ullman's assays employ two different antibodies labeled with either fluorescer or quencher molecules for specific and simultaneous immunological reaction with the ligand to be detected. Thus, the ligand must be polyvalent in nature so as to provide the necessary multiple binding sites for the attachment of multiple antibodies. With increasing concentrations of ligand, more opportunities are provided for the attachment of antibodies, more fluorescence is quenched and a lower level of fluorescence exhibited.

Alternately, and in the case of monovalent ligands, Ullman teaches the use of a ligand analog, the substantial proportion of which has the same spatial and polar organization as the ligand to define one or more determinant or epitopic sites capable of competing with the ligand for the binding sites of the receptor or antibody. The ligand analog differs from the ligand in the absence of an atom or functional group at the site of binding or in having a linking group which has been introduced in place of one or more atoms originally present in the ligand. Typically then, this ligand analog is labeled with either the fluorescer or quencher molecule and competes with the ligand of the sample for the binding site on an antibody which is labeled with the other molecule of the fluorescer-quencher pair. In this mode, increasing concentrations of ligand may be expected to compete more effectively for the binding sites thereby displacing labeled ligand analogs and reducing the amount of fluorescent quenching they would otherwise contribute. Ullman's assays disadvantageously require polyvalency of the ligand or analyte to be detected or in the alternative, disadvantageously require the production of purified intact ligand analogs to compete with the ligand. As already mentioned the production of such substances is a generally disadvantageous, difficult, time consuming and expensive proposition.

It is an object of the present invention to provide a sensitive immunoassay which is equally useful for monovalent or polyvalent analytes and which, in either case, does not require the expensive and difficult production of purified intact analyte-like substances as does Ullman.

It is yet another object of the present invention to employ at least two fluorophores, one of which upon excitation exhibits an emission spectrum capable of exciting the second fluorophore which in turn exhibits an emission spectrum measurably detectable and distinguishable from the first emission spectrum.

It is still another object of the present invention to avoid reliance upon the competition between two immunologically similar antibodies for a single antigenic binding site.

It is a related object to provide an immunoassay which is capable of revealing information concerning the structure of the analyte to be detected and the structure of the corresponding binding site of the antibody.

## Brief Summary of the Invention

In accordance with the principles and objects of the present invention, there are provided immunoassays which, in their most preferred form, employ idiotypic and anti-idiotypic antibodies, each of which is labeled with one or the other of a donor or acceptor molecule of a fluorescent energy transfer pair. The anti-idiotype antibody is advantageously selected to either block or partially block the analyte binding site on the antibody thereby preventing or impeding immunological reaction between the analyte and the antibody. Upon the immunological reaction between the antibody and the anti-idiotype antibody, the acceptor and donor molecules are brought together in close proximity whereby energy transfer may take place pursuant to the Forster dipole-dipole interactions. By exciting the immunoassay reaction mixture, and specifically the donor molecule at its excitation wavelength and measuring the acceptor emission wavelength, one may determine the qualitative or quantitative presence of analyte in the sample.

## Brief Description of the Drawings

Further understanding of the invention may be had by reference to the drawings wherein:

FIGURE 1 diagrammatically depicts an immunological assay based on the analyte, anti-analyte antibody, anti-idiotype antibody and acceptor-donor molecule interactions; and

FIGURES 2 and 3 graphically depict data obtained pursuant to Example 1.

## Detailed Description of the Drawings and Best Mode

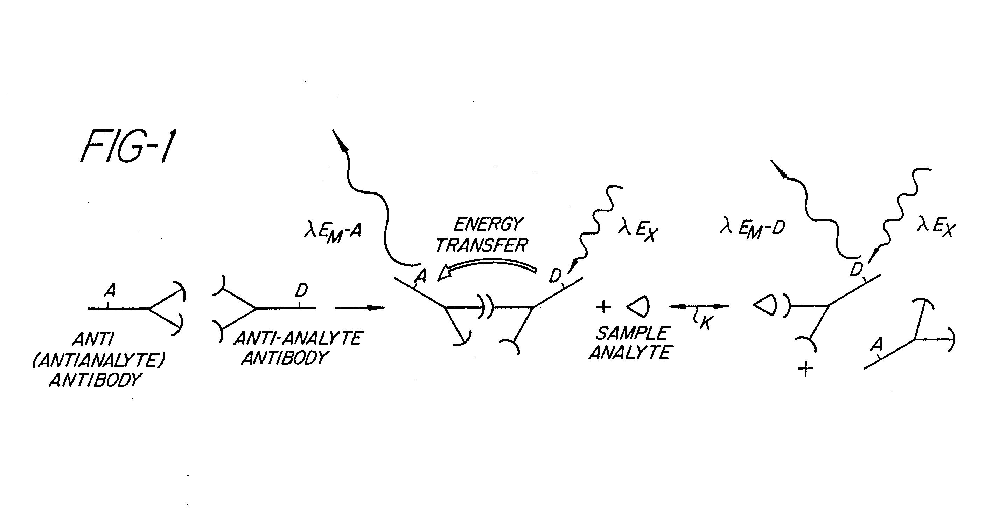

A generalized graphical description of the preferred embodiment of the present invention is shown in Figure 1. Three separate components comprise the reagents and include the sample analyte, a binding partner specific for the sample analyte designated in the diagram as an anti-analyte antibody and the anti-idiotype binding partner reactive with the anti-analyte binding partner and designated as the anti-(anti-analyte)antibody. It will be readily understood by those skilled in the art that the graphical depictions shown are merely artful representations and that actual binding sites of several or more amino acids are more complex in structure and orientation but which complexity is not necessary or integral to an understanding of the instant invention. Pursuant to Figure 1, the anti-analyte antibody is labeled with the donor molecule and the anti-idiotype antibody is labeled with the acceptor molecule, both molecules together comprising a fluorescence energy transfer pair. Again, it will be readily understood that the binding partner-label associations may be readily reversed without significantly altering the assay.

Assuming for the moment an absence of sample analyte, the anti-idiotype and the anti-analyte antibody combine immunologically thereby bringing the acceptor and donor molecules within close physical proximity. Because the energy transfer varies according to the 6th inverse power of the distance, this distance will preferably be regulated by utilizing appropriate labeling techniques of the antibodies so that the acceptor-donor molecules will be closer than 100 angstroms and preferably less than 50 angstroms apart. By exciting the donor, via excitation wavelength lambda-EX (see Figure 1), the donor molecule, by accepting photon energy, is forced to occupy a higher energy level. This energy is released via two mechanisms. Some energy is released by photon emission, and the remainder, depending upon the distance to the acceptor molecule and relative orientation of the acceptor molecule, by an energy transfer mechanism such as the quantum mechanical model described in Biophysical Chemistry, part II: Techniques for the Study of Biological Structure and Function, p. 448ff., by C.R. Cantor and P.R. Shimmel (W. H. Freeman & Co., 1980, New York). The thusly energized acceptor molecule itself returns to its normal energy level via fluorescence emission, designated in Figure 1 as lambda-EM$_a$. Either the fluorescence emission of the acceptor molecule or the fluorescence emission of the donor molecule may be monitored.

Upon addition of the sample analyte in the description of Figure 1, competitive displacement of the anti-idiotype antibody takes place and is governed by equilibrium constant K. The amount of displacement will vary according to the relative affinities between the anti-idiotype antibody and the sample analyte for the binding site on the anti-analyte antibody as well as the relative concentrations thereof. As will be readily apparent to those skilled, these affinities and concentrations may be easily manipulated in order to optimize the sensitivity of the assay. Further, the order of reaction need not be necessarily that shown in Figure 1, conveniently chosen for clarity. Instead it may be preferred to mix reactants simultaneously, or add anti-idiotype antibody after sample analyte has been permitted to immunologically react with the anti-analyte antibody. In any case, the decrease in acceptor emission or the increase in donor emission can be related to the presence of sample analyte. Although qualitative assays are contemplated, a preferred assay will involve the comparison of results with known standards so that quantitative determinations regarding the presence of analyte in the sample may be made. These techniques, will be readily appreciated by those skilled in the art.

In order to enhance clarity, the immunoassay depicted in Figure 1 involved the use of an anti-idiotype antibody capable of pure competition with the analyte for the binding site on the antibody. As has already been previously intimated, the present invention is not so limited, but also contemplates those binding partner variations which act in a manner similar to the basic blocking function of the anti-idiotype antibody. Specifically, anti-framework binding partners or antibodies, antibody fragments and artificially produced peptide analogs are also contemplated. Anti-framework antibodies are those which may react with only a portion of the binding site on the antibody or indeed, may react with no portion of the binding site but instead the so-called framework or contiguous structure associated with or next to the binding site. Thus, the anti-framework antibody although not binding with the analyte binding site, prevents the binding of the analyte because of steric hindrance or other protein conformational changes. It is the possibility of providing such anti-idiotype, anti-framework, antibody fragments or the like binding partners alone and/or in combination with the energy transfer orientation component, that will enable one to study either the structure of related analytes, or the spatial configurations and structure of the antibody binding sites themselves.

The actual production of antibodies immunologically reactive to antigens, haptens or analytes generally is well-known, particularly with respect to antibodies of polyclonal origin. Pursuant to the hybridoma generation principles and methods described by Kohler and Milstein (Nature Vol. 256, p. 495, 1975) and others, the production of monoclonal antibodies, their selection and characterization has also become standard practice in this art. By employing the generated antibodies as the antigenic substance, anti-idiotype, anti-framework and the like antibodies may also be readily produced. For example, the production of anti-idiotypic anti-arsenate antibodies is described by Kuettner et al., "Quantitative Investigation of Idiotypic Antibodies VI, Idiotypic Specificity as a Potential Genetic Marker for the Variable Regions of Mouse Immunoglobulin Polypeptide Chains", J. Exp. Med. 135:579 (1972). The procedures described therein were used to produce the anti-idiotype antibodies employed in Example 1 described below. It will be readily apparent that the procedures described therein can be suitably modified to readily permit the production of virtually any anti-idiotype or similar antibody. Likewise antibodies may be raised by using only critical peptide sequences instead of the whole

- 11 -

0150905

protein as these sequences may, in some circumstances, be more
easily produced under controlled conditions.

As with all fluorescence immunoassays, the presence of nonspecific
background fluorescence only serves to degrade the signal to noise
ratio and thus decrease sensitivity. Preferably, background
fluorescence will be minimized or eliminated altogether by the
judicious choice of buffers and reagents as well as the careful
handling thereof. Further, the choice of fluorescent energy
transfer pairs will significantly affect the size of this
nonspecific noise component in the detected fluorescent signal. For
instance, it is well-known that background fluorescence decreases
significantly with increases in wavelength. Accordingly, by
employing an acceptor molecule which emits in the far red or near
infrared region of the spectrum, the signal to noise ratio may be
suitably optimized thereby enhancing sensitivity.

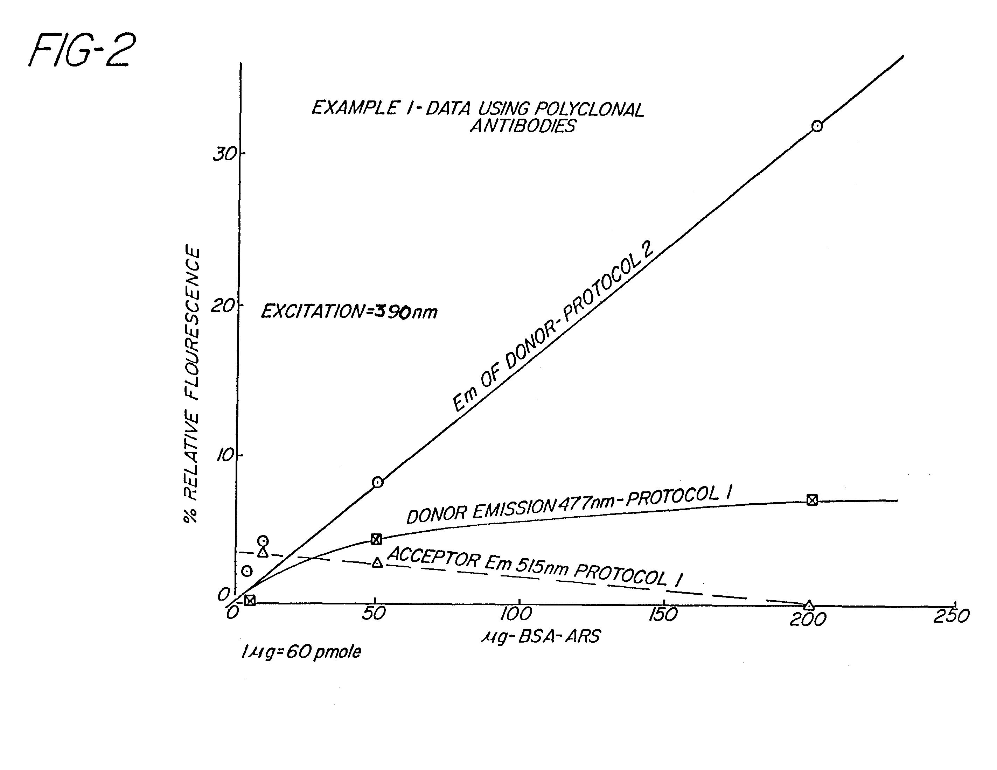

Further understanding of these and other principles of the instant
invention may be had by studying Example 1 which describes an
anti-idiotype fluorescence energy transfer immunoassay, the results
of which have been graphically depicted in Figure 2.

Still other alternatives are contemplated by the instant invention
concerning the antibodies, or more broadly the binding partners
which are immunologically specifically reactive with the analyte to
be determined. As already mentioned, the binding partners may be
antibodies of polyclonal origin or, of monoclonal origin, the latter
generally having less nonspecific reactivity as well as having more
easily selected binding affinities. It may further be found
desirable to employ antibody fragments such as F(ab) or F(ab)$_2$'
portions in order to optimize sensitivity. Specifically, selection
of Fab' antibody fragments may be expected to eliminate multiple
binding problems whereby the analyte specific antibody may be bound
simultaneously to analyte as well as anti-idiotype antibody due to
the presence of multiple binding sites on complete antibodies.
These latter immunological complexes may be expected to reduce sensitivity when compared to immunoassays employing reagents such as the analyte specific, antibody fragment which is capable of binding either the analyte or the anti-idiotype antibody but not both. Other types of antibody fragments which are not functionally significantly different from the foregoing as functionally equivalent peptide sequences are equally contemplated.

Example 1

Idiotype-Anti-Idiotype Fluorescence Energy Transfer Immunoassay for Bovine Serum Albumin-Azobenzenearsonate

In accordance with the principles of the present invention, the assay is based on the competitive binding of the antigen, bovine serum albumin-azobenzenearsonate, BSA-Ars, and the anti-idiotype antibody, anti-(anti-Ars), towards the anti-bovine serum albumin-azobenzenearsonate antibody, anti-Ars. The dye pair used for fluorescence energy transfer is the donor-acceptor pair fluorescamine (FCA)-dichlorotriazinyl fluorescein (DTAF). FCA can be excited at 390 nm and exhibits an emission peak maximum at 477 nm which overlaps with the excitation peak of DTAF which centers around 495 nm. When FCA and DTAF are within energy transfer distance, preferably about 50 A or less, the observed emission of FCA is substantially reduced due to energy transfer to DTAF. Thus, there will be a decrease of 477 nm emission from FCA accompanied by an increase of fluorescence emission at 515 nm from DTAF.

When the anti-(anti-Ars) is labeled with DTAF and the anti-Ars is coupled to FCA, the amount of immunological binding can be monitored by the change of emission at either 477 nm or 515 nm before and after mixing the components. By increasing the BSA-Ars concentration in the mixture (thereby displacing acceptor molecules), and monitoring the increase in emission of the donor at 477 nm or the decrease in emission of the acceptor at 515 nm, a standard curve proportional to increased antigen concentration (and increased antigen-antibody immunological reaction) can be

CRL-5

established permitting quantitative evaluation of the presence of BSA-Ars in an aqueous sample. As may be readily appreciated, qualitative determinations may also be made by merely detecting changes in fluorescent emission upon addition of the sample to the reactants. Fluorescent emissions may be measured with virtually any type of standard fluorescence spectrophotometer.

(II) Materials

All reagents were used without purification except as otherwise indicated. FCA and DTAF were obtained from Sigma Chemical Co. The BSA-Ars, anti-Ars, and anti-(anti-Ars) were prepared in accordance with well-known techniques as described previously.

(III) Preparation of Reagents

1. The (anti-Ars)-FCA conjugate was prepared by incubating 1 mg of anti-Ars suspended in 1 ml of reagent buffer with 5 mg of FCA in 1CO µl acetone solution for 15 min at room temperature. The mixture was fractionated through a DEAE-Sephacel ion-exchange column with a sodium chloride gradient. The change of fluorescence at 477 nm and protein UV absorption at 280 nm were recorded for all fractions and those corresponding to the protein-dye conjugate were pooled and stored at $4^{\circ}$ C with 0.1% sodium azide buffer.

2. To couple the DTAF to the anti-(anti-Ars) antibody, a buffer solution containing 0.5 mg of anti-(anti-Ars) was incubated with 1.5 mg of DTAF for 1 hr at room temperature. Isolation of the conjugate was performed by following the same procedure described for the isolation of the (anti-Ars)-FCA.

3. The assay buffer was formulated to contain 0.1 M NaCl, 0.005 M borate buffer at pH 8, 0.1% sodium azide, and 1% polyethylene glycol.

-5

4. The reagent buffer used in the labeling preparation steps 1 and 2 was a borate saline buffer prepared with 0.15 M NaCl and 0.015 mM borate adjusted to pH 8.

(IV) Assay Conditions

Two protocols were used to establish the standard curve for the assay of BSA-Ars. The protocols differed in the order in which reactants and sample were reacted.

Protocol (1): One Step Assay

0.3 ml of (anti-Ars)-FCA and 0.3 ml of anti-(anti-Ars)-DTAF were mixed with a series of 0.1 ml of BSA-Ars samples having concentrations ranging from 0.5 µg to 200 µg. The resultant mixtures were incubated at 37° C for one hour. The incubated mixtures were excited at 390 nm and fluorescence emission at both 477 nm and 515 nm measured. The fluorescence of the BSA-Ars and the buffer was also recorded for subsequent standard background fluorescence correction.

Protocol (2): Sequential Assay

0.3 ml of (anti-Ars)-FCA and a series of 0.1 ml of BSA-Ars samples having concentrations ranging from 0.5 µg to 200 µg were incubated at 37° C for one hour. 0.3 ml of anti-(anti-Ars)-DTAF was then added and the mixture incubated for an additional 30 minutes at room temperature. The incubated mixtures were excited at 390 nm and fluorescence at 477 nm measured.

(V) Results and Data

In both assays, the fluorescence contribution of BSA-Ars and the buffer was subtracted from observed fluorescence to obtain corrected fluorescence in accordance with standard techniques. The data obtained is presented in FIG. 2.

CRL-5

(VI) Discussion

The increase of donor fluorescence at 477 nm and the concurrent decrease of acceptor fluorescence emission at 515 nm with increasing concentration of BSA-Ars is consistent with the expected decrease in energy transfer due to the reduced availability of antibody binding sites for the anti-idiotype antibody.

Example 2

Idiotype-Anti-Idiotype Fluorescence Energy Transfer Immunoassay for Bovine Serum Albumin-Azobenzenearsonate Using Monoclonal Antibodies

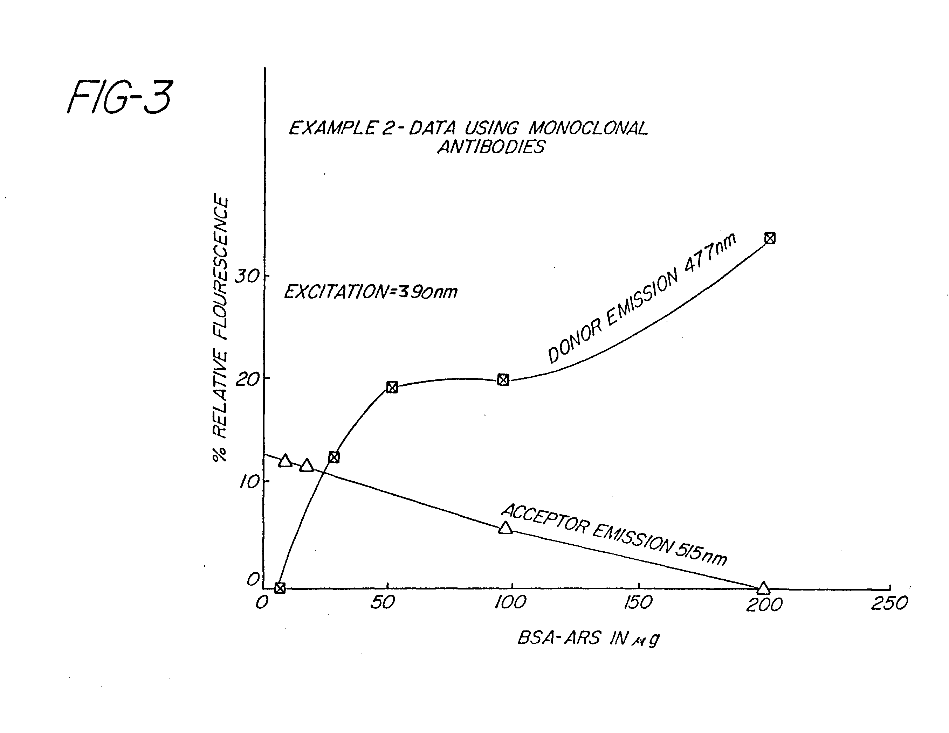

Using procedures described previously and those well-known in the art, monoclonal antibodies were raised to replace each of the polyclonal antibodies employed in Example 1 (i.e. monoclonal anti-Ars antibody and monoclonal anti-(anti-Ars) antibody, the latter being the anti-idiotype antibody). Protocol 2, the sequential assay, described in Example 1, was repeated with these reagents and the emission spectra of the acceptor and donor molecules measured over various ranges of analyte concentration. The data obtained is presented in Figure 3. As expected, an .increase in sensitivity was observed due to the replacement of polyclonal antibodies with monoclonal antibodies and the concommittant elimination of nonspecific reactions.

It should be noted that one skilled in the art will recognize that various improvements to the assay can be readily made to increase sensitivity such as the employment and selection of monoclonal antibodies to reduce nonspecific binding and regulate antibody avidity and affinity, or the measurement of the changes in time rate constants of donor emission as a measurement of binding and analyte presence without departing from the spirit or scope of the present invention.

CRL-5

0150905

What is Claimed is:

1.   An immunoassay for detecting an analyte comprising:

a first binding partner immunologically reactive with said analyte;

a second binding partner immunologically reactive with said first binding partner and whose spatial relationship to said first binding partner is altered upon immunological reaction of said analyte with said first binding partner; and

means associated with said first and second binding partners for providing a detectable signal responsive to said altered spatial relationship.

2.   The immunoassay as claimed in Claim 1 wherein said associated means comprises a fluorescent energy transfer pair having a donor molecule with a first excitation wavelength and a first emission wavelength and an acceptor molecule capable of being excited by said first emission wavelength and having a second emission wavelength distinguishably detectable from said first emission wavelength.

3.   The immunoassay as claimed in Claim 2 wherein one of said donor or acceptor molecules is associated with the first binding partner and the other of said molecules is associated with the second binding partner.

4.      The immunoassay as claimed in any one of claims 1 to 3 wherein said first and second binding partners are selected from the group consisting of polyclonal antibodies, monoclonal antibodies, F(ab)' antibodies, F(ab) antibodies and antibody fragments.

5. The immunoassay as claimed in Claim 4 wherein said second binding partner is selected from the group consisting of anti-idiotype antibodies and anti-framework antibodies.

6. The immunoassay as claimed in any one of Claims 1 to 5 wherein said responsive detectable signal is selected from the group consisting of increased or decreased donor fluorescent emission, increased or decreased acceptor fluorescent emission, increased or decreased donor emission time constant, and any combination of the foregoing.

7. An immunoassay for detecting an immunologically reactive analyte or for determining the structure thereof wherein the binding of said analyte to an antibody having an idiotype changes the spatial relationship between said antibody and an anti-idiotype antibody capable of binding said idiotype, said change in spatial relationship or relative motion being detectable by fluorescent means.

8. A homogeneous immunoassay for detecting an analyte, having an immunologically reactive antigenic determinant, in response to the change in spatial relationship between 1) an antibody specific for the antigenic determinant and characterized with an idiotype or 2) a framework and an anti-idiotype or anti-framework antibody.

9. The immunoassay as claimed in Claim 7 or Claim 8 wherein the changes are detected by measuring the change in fluorescence emission or the change in fluorescence emission time constant.

10. The immunoassay as claimed in any one of claims 7 to 9 wherein the fluorescent means comprises a fluorescent energy transfer pair.

11. The immunoassay as claimed in Claim 10 wherein the fluorescent energy transfer pair comprises a donor molecule having a first excitation wavelength and a first emission wavelength and an acceptor

-18-

molecule capable of being excited by said first emission wavelength and having a second wavelength distinguishably detectable from said first emission wavelength.

12. The immunoassay as claimed in any one of claims 7 to 11 wherein at least one of the antibodies is an incomplete antibody.

13. An immunoassay method for analyzing for the presence of an analyte in a sample for use with a detectable label system comprising a fluorescent energy transfer pair having a donor molecule with a first excitation wavelength and a first emission wavelength and an acceptor molecule capable of being excited by said first emission wavelength and having a second emission wavelength, distinguishably detectable from said first emission wavelength, said assay method comprising the steps of:

providing the sample containing the analyte to be analyzed;

further providing a first binding partner specific for the analyte and labeled with either the acceptor or the donor molecule of the acceptor-donor pair;

forming a first mixture by contacting said first binding partner with a second binding partner specific for said first binding partner and labeled with the other of said donor or acceptor molecule under conditions which allow any immunological reaction to occur, said second binding partner having a spatial relationship with said first binding partner which can be altered upon immunological reaction of said analyte with said first binding partner;

contacting said sample with said first mixture to form a second mixture;

incubating said second mixture under conditions whereby immunological binding of said analyte with said first binding partner is permitted to occur;

irradiating said second mixture with light having a wavelength capable of exciting said donor molecule but not having a wavelength capable of substantially exciting said acceptor molecule; and

detecting either the decrease in the first fluorescence emission or the increase in the second fluorescence emission and determining the presence of said analyte on the basis of said decrease or increase in fluorescence.

14. An immunoassay method for analyzing for the presence of an analyte in a sample for use with a detectable label system comprising a fluorescent energy transfer pair having a donor molecule with a first excitation wavelength and a first emission wavelength and an acceptor molecule capable of being excited by said first emission wavelength and having a second emission wavelength, said assay method comprising the steps of:

contacting the sample containing the analyte to be measured with a first binding partner specific for the analyte and labeled with either the acceptor or the donor molecule of the acceptor-donor pair under conditions permitting any immunological reaction to occur;

further contacting said sample and said first binding partner with a second binding partner, specific for said first binding partner and labeled with the other of said donor or acceptor molecule under conditions whereby said second binding partner competes with said analyte for binding sites on said first binding partner;

irradiating said second mixture with light having a wavelength capable of exciting said donor molecule but not having a wavelength capable of substantially exciting said acceptor molecule; and

detecting either the decrease in the first fluorescence emission or the increase in the second fluorescence emission and determining the presence of said analyte on the basis of said decrease or increase in fluorescence.

15. An immunoassay method for analyzing for the presence of an analyte in a sample for use with a detectable label system comprising a fluorescent energy transfer pair having a donor molecule with a first excitation wavelength and a first emission wavelength and an acceptor molecule capable of being excited by said first emission wavelength and having a second emission wavelength distinguishably detectable from said first emission wavelength, said assay method comprising the steps of:

mixing in a single step:

the sample containing the analyte to be measured, a first binding partner specific for the analyte and labeled with either the acceptor or the donor molecule of the acceptor-donor fluorescent energy transfer pair; and a second binding partner specific for said first binding partner and labeled with the other of said donor or acceptor molecule under conditions which allow any immunological reaction to occur whereby binding of the analyte to said first binding partner alters the spatial relationship between said first and second binding partners;

irradiating said second mixture with light having a wavelength capable of exciting said donor molecule but not having a wavelength capable of substantially exciting said acceptor molecule; and

detecting either the decrease in the first fluorescent emission or the increase in the second fluorescent emission and determining the presence of said analyte on the basis of said decrease or increase in fluorescence.

16. The method as claimed in any one of claims 13 to 15 wherein said determining step further comprises comparing said increase or decrease to a standard curve whereby the quantity of said analyte in said sample may be determined.

17. The method as claimed in any one of claims 13 to 15 wherein said detecting step comprises determining the donor fluorescent time constant and determining the presence of said analyte on the basis of changes in said time constant.

18. The method as claimed in any one of claims 13 to 17 wherein said first and second binding partners are selected from the group consisting of polyclonal antibodies, monoclonal antibodies, F(ab) antibodies, $F(ab)_2'$ antibodies and antibody fragments.

19. The method as claimed in claim 18 wherein one or both of said binding partners are monoclonal antibodies.

20. Reagent kit for use in a competitive homogeneous immunoassay system for an analyte using fluorescent energy transfer between a donor whose emission is capable of exciting an acceptor molecule having a fluoresce of emission distinguishable from the donor emission and where the excitation wavelength employed to excite the donor molecule results in no appreciable excitation of the acceptor molecule, said kit comprising:

a first binding partner specific for the analyte to be assayed and labeled with either the donor molecule or the acceptor molecule of the donor-acceptor fluorescent energy transfer pair; and

a second binding partner labeled with the other of said donor or acceptor molecule of said donor-acceptor fluorescent energy transfer pair, said second binding partner capable of reacting with said first binding partner and whose spatial relationship with said first binding partner is altered upon immunological reaction between said analyte to be determined and said first binding partner.

CRL-5

FIG-1

$\lambda E_M$-$A$    ENERGY TRANSFER    $\lambda E_X$    $\lambda E_M$-$D$    $\lambda E_X$

$A$    $D$    $D$

ANTI (ANTIANALYTE) ANTIBODY    ANTI-ANALYTE ANTIBODY    SAMPLE ANALYTE    $A$

$K$

FIG-2

EXAMPLE I - DATA USING POLYCLONAL ANTIBODIES

EXCITATION = 390nm

Em OF DONOR - PROTOCOL 2

DONOR EMISSION 477nm - PROTOCOL I

ACCEPTOR Em 515nm PROTOCOL I

% RELATIVE FLOURESCENCE

µg - BSA - ARS

1µg = 60 pmole

2/3

0150905

FIG-3

EXAMPLE 2 - DATA USING MONOCLONAL ANTIBODIES

EXCITATION = 390nm

DONOR EMISSION 477nm

ACCEPTOR EMISSION 515nm

% RELATIVE FLOURESCENCE

BSA-ARS IN μg

3/3

0150905